**Europäisches Patentamt**

**European Patent Office**

**Office européen des brevets**

(11) Veröffentlichungsnummer: **0 446 399 A1**

## EUROPÄISCHE PATENTANMELDUNG

(21) Anmeldenummer: **90104987.4**

(22) Anmeldetag: **16.03.90**

(51) Int. Cl.5: **A61K 31/095, A61K 9/06**

(43) Veröffentlichungstag der Anmeldung:
**18.09.91 Patentblatt 91/38**

(84) Benannte Vertragsstaaten:
**AT BE CH DE FR GB IT LI NL SE**

(71) Anmelder: **ADATOMED PHARMAZEUTISCHE UND MEDIZIN-TECHNISCHE GESELLSCHAFT MBH**
**Am Moosfeld 27**
**W-8000 München 82(DE)**

(72) Erfinder: **Kaden, Peter**
**Hans-Böckler-Allee 55**
**W-5100 Aachen(DE)**

(74) Vertreter: **Nöth, Heinz, Dipl.-Phys. et al**
**Patentanwälte Pfenning, Meinig & Partner**
**Mozartstrasse 17**
**W-8000 München 2(DE)**

(54) **Zellwachstum hemmende Lösung.**

(57) Eine Epithelzellwachstum hemmende Lösung zur Verhinderung von Nachstarbildung nach einer Kataraktoperation mit einem Gehalt an höchstens 50 % Ethanol und höchstens 0,1 5 n-Dodecylsulfat-Natriumsalz.

**EP 0 446 399 A1**

Nach extrakapsulären Kataraktoperationen, bei denen Augenlinsen durch künstliche Intraokularlinsen ersetzt werden, kommt es häufig zur Nachstarbildung. Diese Nachstarbildung ist zurückzuführen auf in der Linsenkapsel verbleibende Epithelzellen. Diese Epithelzellen lassen sich nach Entfernen der natürlichen Augenlinse aus der Linsenkapsel in aller Regel nicht erkennen, vor allem, wenn sie im äquatorialen Bereich, der durch die Iris abgedeckt ist, liegen. Selbst wenn man diese noch verbliebenen Epithelzellen erkennt, würde sich eine Entfernung nur schwierig gestalten. Verbleiben solche Epithelzellen nach der Implantation der künstlichen Intraokularlinse in der Linsenkapsel, kommt es durch Proliferation dieser Epithelzellen zu Nachstarbildung.

Aufgabe der Erfindung ist es, eine Möglichkeit zu schaffen, mit der die Nachstarbildung nach einer Katarakoperation wirksam verhindert wird.

Diese Aufgabe wird erfindungsgemäß durch eine Epithelzellwachstum hemmende Lösung gelöst, welche gekennzeichnet ist durch einen Gehalt an höchstens 50 % Ethanol, insbesondere 30 % Ethanol, und höchstens 0,1 % n-Dodecylsulfat-Natriumsalz in destilliertem Wasser, bevorzugt bidestilliertem Wasser (Aqua bidest.).

Eine 20-Sekunden-Inkubation der Epithelzellen mit dieser Lösung bewirkt einen 100 %igen Zellschaden dahingehend, daß die Zellen morphologisch degeneriert sind und an der Stoffwechselaktivität nicht mehr teilnehmen. Die Linsenkapsel wird dabei jedoch nicht beschädigt. Dies ist auch darauf zurückzuführen, daß - wie sich gezeigt hat - durch den Epithelzellrasen die Kapsel während der Behandlung mit der Lösung abgedeckt bleibt und mit der Lösung nicht in Berührung kommt. Auf diese Weise lassen sich die bei einer Kataraktoperation in der Linsenkapsel noch verbliebenen Epithelzellen irreversibel degenerieren, ohne daß die Linsenkapsel angegriffen wird. Man erreicht hierdurch eine wirksame Verhinderung der Nachstarbildung.

Es genügt eine Inkubation der Zellen mit der Lösung von 20 Sekunden, um eine 100 %ige irreversible Degenerierung der Zellen zu erreichen. Die Lösung kann bis zu 20 % insbesondere mit einer Phosphatpufferlösung verdünnt sein. Es läßt sich dann ebenfalls noch eine 100 %ige Wirkung erzielen. Die Inkubationszeit für die Epithelzellen wird dann etwa auf 30 Sekunden bemessen. Eine Erhöhung der Konzentration von n-Dodecylsulfat über 0,1 kann eine Gefahr des Analysierens der Linsenkapsel bedeuten. Bevorzugt wird eine Konzentration von 0,08 % n-Dodecylsulfat-Natriumsalz.

Für die irreversible Schädigung der Epithelzellen in einer Augenlinsenkapsel hat sich als optimale Lösung eine 20 %ige Verdünnung bevorzugt mit PBS der oben angegebenen Lösung, welche 30 % Ethanol und 0,1 % n-Dodecylsulfat-Natriumsalz in Aqua bidest. enthält, erwiesen.

Ein bevorzugtes Ausführungsbeispiel der mit dem Phosphatpuffer verdünnten Lösung beinhaltet

|  | mg/l |
|---|---|
| n-Dodecylsulfat Natriumsalz | 800 |
| NaCl | 1600 |
| KCl | 40 |
| Na₂HPO₄ x 2 H₂O | 288 |
| KH₂PO₄ | 40 |
| Ethanol pA 300 ml | |
| Aqua bidest. ad 1000 ml | |

Die angegebenen Substanzen werden in ca. 500 ml Aqua dest. gelöst, und es erfolgt dann die Zugabe von Ethanol. Schließlich wird ad 1000 Aqua dest. aufgefüllt. Bevorzugt kommt Aqua bidest. zum Einsatz.

Die das Epithelzellwachstum hemmende Lösung kann mit Hilfe einer in die Linsenkapsel einsetzbaren Spritze in die Linsenkapsel, aus welcher die Augenlinse entfernt ist, eingebracht werden. Die das Epithelzellwachstum hemmende Lösung kommt während einer relativ kurzen Zeit von etwa 20 bis 30 Sekunden zur Einwirkung, während welcher die in der Linsenkapsel verbliebenen Linsenepithelzellen so weit geschädigt werden, daß eine weitere Proliferation verhindert wird. Anschließend wird durch die zum Behandlungssystem gehörige Spritze eine isotonische, an das Kammerwasser ange paßte Neutralisationslösung der Spüllösung zur Beseitigung der das Epithelzellwachstum hemmenden Lösung eingebracht.

Es ist auch möglich, die Zellwachstum hemmende Lösung im Rahmen eines Behandlungssystems durch einen Träger für die das Epithelzellwachstum hemmdende Lösung zu ergänzen. Bei diesem Träger handelt es sich bevorzugt um eine viskoela stische Lösung, wie sie zur Volumensubstitution des bei der Öffnung der Vorderkammer ausgeflossenen Kammerwassers zur Aufrechterhaltung einer tiefen Vorderkammer während des intraokularen Eingriffes und zum Schutz des hochempfindlichen intraokularen Gewebes gegen unmittelbaren Kontakt der chirurgischen Instrumente verwendet wird. Es kann sich hier um eine diesbezüglich bekannte zweiprozentige Hydroxypropylmethylcellulose-Lösung(Adatocel) oder auch um eine zu diesem Zweck bekannte Hyaluronsäurelösung (Healon) handeln. Auch ein formstabiles Gel ist geeignet.

Im Rahmen eines Behandlungssystems kann auch die Neutralisationslösung, insbesondere zur Nachspülung, als bevorzugt an das Kammerwasser angepaßte sterile isotonische Salzlösung verwendet werden.

Ein bevorzugtes Ausführungsbeispiel für ein Neutralisationsmittel ist folgende Lösung:

Sterile isotone Lösung

Zusammensetzung: 1 ml enthält

| | |
|---|---|
| Natriumchlorid | 6,4 mg |
| Kaliumchlorid | 0,75 mg |
| Calciumchlorid . 2 $H_2O$ | 0,48 mg |
| Magnesiumchlorid . 6 $H_2O$ | 0,3 mg |
| Natriumacetat . 3 $H_2O$ | 3,9 mg |
| Natriumcitrat . 2 $H_2O$ | 1,7 mg |

Die Epithelzellwachstum hemmende Lösung kommt bevorzugt in einem Behandlungssystem zur Verhinderung von Nachstarbildung nach einer Kataraktoperation bzw. nach dem Entfernen der natürlichen Augenlinse aus der Linsenkapsel zum Einsatz.

**Patentansprüche**

1. Zellwachstum hemmende Lösung, gekennzeichnet durch einen Gehalt an höchstens 50 % Ethanol und höchstens 0,1 % n-Dodecylsulfat in destilliertem Wassser.

2. Lösung nach Anspruch 1, gekennzeichnet durch einen Gehalt an 30 % Ethanol und höchstens 0,1 % n-Dodecylsulfat in destilliertem Wasser.

3. Lösung nach Anspruch 1 oder 2, gekennzeichnet durch einen Gehalt an 0,08 % n-Dodecylsulfat.

4. Lösung nach einem der Ansprüche 1 bis 3, gekennezeichnet durch eine 20 %ige Verdünnung.

5. Lösung nach Anspruch 4, gekennzeichnet durch eine Verdünnung mit einer Phosphatpufferlösung.

6. Lösung nach einem der Ansprüche 1 bis 5, gekennzeichnet durch

| | mg/l |
|---|---|
| n-Dodecylsulfat Natriumsalz | 800 |
| NaCl | 1600 |
| KCl | 40 |
| $Na_2HPO_4$ x 2 $H_2O$ | 288 |
| $KH_2PO_4$ | 40 |
| Ethanol pA 300 ml | |
| Aqua bidest. ad 1000 ml | |

3

7. Verwendung einer Lösung nach einem der Ansprüche 1 bis 5 als Epithelzellenwachstum hemmende Lösung.

8. Verwendung einer Lösung nach einem der Ansprüche 1 bis 6 als wachstumhemmende Lösung für Epithelzellen in der Kapsel einer Augenlinse.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | WO-A-8 908 474 (ALLERGAN INC.) * Ansprüche; Seite 1; Seite 2, Zeilen 1-11 * | 1-7,8 | A 61 K 31/095 A 61 K 9/06 |
| A | EP-A-0 077 197 (BAYLOR COLLEGE OF MEDICINE, TEXAS) * Ansprüche; Seite 1, Zeilen 1-20 * | 1-7,8 | |
| A | EP-A-0 299 467 (OPHTHALMIC RESEARCH) * Ansprüche; Seite 2, Zeilen 5-8,25-30 * | 1-7,8 | |

RECHERCHIERTE
SACHGEBIETE (Int. Cl.5)

A 61 K

Der vorliegende Recherchenbericht wurde für alle Patentansprüche erstellt

| Recherchenort | Abschlußdatum der Recherche | Prüfer |
|---|---|---|
| DEN HAAG | 14-09-1990 | NAUCHE S.A. |